(19) Europäisches Patentamt
European Patent Office
Office européen des brevets

(11) **EP 2 760 398 B1**

(12) **EUROPÄISCHE PATENTSCHRIFT**

(45) Veröffentlichungstag und Bekanntmachung des Hinweises auf die Patenterteilung:
**01.05.2019 Patentblatt 2019/18**

(21) Anmeldenummer: **12769361.2**

(22) Anmeldetag: **24.09.2012**

(51) Int Cl.:
***A61F 9/007*** *(2006.01)*

(86) Internationale Anmeldenummer:
**PCT/EP2012/068767**

(87) Internationale Veröffentlichungsnummer:
**WO 2013/045394 (04.04.2013 Gazette 2013/14)**

(54) **OPHTHALMOCHIRURGISCHE STEUERUNGSVORRICHTUNG**

OPHTHALMO-SURGICAL CONTROL DEVICE

DISPOSITIF DE COMMANDE OPTHALMOCHIRURGICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.09.2011 DE 102011114584**

(43) Veröffentlichungstag der Anmeldung:
**06.08.2014 Patentblatt 2014/32**

(73) Patentinhaber: **Carl Zeiss Meditec AG**
**07745 Jena (DE)**

(72) Erfinder: **EICHLER, Michael**
**73434 Aalen (DE)**

(74) Vertreter: **Carl Zeiss AG - Patentabteilung**
**Carl-Zeiss-Strasse 22**
**73447 Oberkochen (DE)**

(56) Entgegenhaltungen:
**EP-A1- 1 849 444      EP-A1- 1 990 032**
**WO-A2-2010/054144**

## Beschreibung

Die Erfindung betrifft eine ophthalmochirurgische Steuerungsvorrichtung und ein ophthalmochirurgisches System mit einer derartigen Steuerungsvorrichtung.

Die Erfindung ist in den Ansprüchen definiert.

Zur Behandlung einer Linsentrübung, welche in der Medizin als grauer Star bezeichnet wird, gibt es mehrere chirurgische Techniken. Die am weitesten verbreitete Technik ist die Phakoemulsifikation. Dabei wird eine eingetrübte Augenlinse mittels einer im Ultraschallbereich schwingenden Hohlnadel, welche an ihrem vorderen Ende eine Schneidspitze aufweist, derartig in kleine Bestandteile zertrümmert (emulsifiziert), dass diese Bestandteile durch die Hohlnadel abgesaugt werden können. Anschließend setzt der Operateur als Ersatz für die derart zertrümmerte Linse eine künstliche Linse ein. Eine wesentliche Baugruppe bei der Durchführung einer solchen Phakoemulsifikation ist ein Handstück, welches die genannte Hohlnadel aufweist. Die erforderlichen Ultraschallschwingungen zur Zertrümmerung der getrübten Augenlinse können derart erzeugt werden, dass das Handstück mit piezokeramischen Elementen versehen ist. Legt man an diese piezokeramischen Elemente eine Spannung an, kann aufgrund des piezoelektrischen Effektes eine Längenänderung bewirkt werden, so dass eine mit den piezokeramischen Elementen verbundene Nadel in Longitudinalrichtung ausgelenkt werden kann.

Aus der EP 1849444 A1 ist ein System zur Steuerung eines Ultraschall-Handstücks mit einem Klasse D Verstärker bekannt.

Aus der EP0765637 ist ein chirurgisches Ultraschallgerät bekannt, das mit einem Überlagerungsmodul additiv mehrere Frequenzen überlagert und damit ein piezoelektrisches Gerät ansteuert.

Aus der WO 2010/054144 A2 ist ein Verfahren und eine Vorrichtung zum Steuern eines Ultraschallhandstückes für eine Phakoemulsifikation bekannt. Das Design des Systems ist auf den Betrieb eines Ultraschallhandstück gerichtet, das longitudinale und transversale Bewegungen ausführen kann.

Um in möglichst kurzer Zeit eine vollständige Emulsifikation der Augenlinse und somit einen hohen Wirkungsgrad zu erreichen, ist es sinnvoll, die Hohlnadel mit möglichst großen Amplituden zu bewegen. Dies lässt sich derart durchführen, dass die piezoelektrischen Elemente im Bereich der Resonanzfrequenz des Handstückes betrieben werden. Die Resonanzfrequenz eines Handstückes mit einer Hohlnadel kann man im lastfreien Zustand sehr genau bestimmen. Sobald die Nadel jedoch in Kontakt mit der zu emulsifizierenden Linse tritt, ändern sich die Massenverhältnisse, so dass sich die Resonanzfrequenz verschiebt. Um jeweils möglichst im Bereich der Resonanzfrequenz ein solches Handstück zu betreiben, wird in US 6,997,935 B2 vorgeschlagen, die Phasenlage zwischen der angelegten Spannung und dem fließenden Strom zum Betreiben der piezoelektrischen Elemente zu erfassen und so zu regeln, dass gemäß der Gleichung $P = U \cdot I \cdot \cos \varphi$ eine möglichst maximale Leistung erzielt wird. Damit der Faktor $\cos \varphi$ einen möglichst hohen Betrag einnimmt, muss $\cos \varphi = 1$ bzw, $\varphi = 0$ betragen. Eine solche Situation ist bei einem Resonanzfall gegeben. Verschiebt sich jedoch die Resonanzfrequenz zum Beispiel aufgrund einer Änderung der mechanischen Belastung der Schneidspitze, ist der Phasenwinkel $\varphi$ nicht gleich 0, sondern liegt in dem Bereich zwischen 0 und $-\pi/2$ bzw. 0 und $+\pi/2$. Gemäß US 6,997,935 B2 wird nach dem Erfassen des Phasenwinkels $\varphi$ die Anregungsfrequenz so geregelt, dass die Anregungsfrequenz der Schneidspitze mit der Eigenfrequenz $\omega_0$ übereinstimmt.

Eine Ursache für die Verlagerung der Resonanzfrequenz ist nicht nur eine veränderte Belastung durch Linsenbruchstücke (Massenänderung), sondern auch Erwärmung des Handstückes bei längerem Betrieb sowie eine Alterung der piezokeramischen Elemente und damit Veränderung ihrer physikalischen Eigenschaften. Diese Parameter können sich in beliebiger Form überlagern. Dies hat den Nachteil, dass für ein Schwingen der piezokeramischen Elemente genau in ihrer Resonanzfrequenz permanent nachgeregelt werden muss. Ein weiterer Nachteil besteht darin, dass zur Bestimmung des Phasenwinkels stets mehrere aufeinander folgende Messpunkte des Spannungs- und Stromverlaufes über die Zeit erfasst werden müssen. Dies bewirkt eine relativ langsame Regelung. Die Folge ist, dass trotz eines solch großen messtechnischen und regelungstechnischen Aufwandes das Handstück nicht wirklich in seiner Resonanzfrequenz schwingt. Vielmehr liegt aufgrund der langsamen Regelung immer eine erhebliche zeitliche Verzögerung in der Anpassung an die jeweilige momentane Resonanzfrequenz vor.

Werden während einer Phakoemulsifikation die piezokeramischen Elemente des Handstückes relativ lange Zeit angesteuert, erwärmen sich nicht nur die piezokeramischen Elemente. Auch der Umgebungsbereich der von den Piezokeramiken angesteuerten Hohlnadel erwärmt sich bei genügend langem Betrieb so sehr, dass die für die Phakoemulsifikation durchstochene Hornhaut in der Umgebung der Nadel verbrennen kann. Da eine solche Verletzung unbedingt vermieden werden muss, ist es üblich, die piezokeramischen Elemente für eine vorbestimmte Zeit in ihrem Betrieb zu unterbrechen. In den so entstehenden Betriebspausen wird den piezokeramischen Elementen keine Ultraschallenergie zugeführt, so dass die Hohlnadel und ihre Umgebung abkühlen können. Nachteilig dabei ist, dass die piezokeramischen Elemente relativ aufwendig angesteuert werden müssen, um während vorbestimmter Zeiten in einem Schwingungszustand oder in einem Pausenzustand sein zu können.

Es besteht daher eine Aufgabe darin, eine ophthalmochirurgische Steuerungsvorrichtung und ein System mit einer solchen Steuerungsvorrichtung zu schaffen, mit der die Emulsifikation einer Augenlinse in kurzer Zeit mit hohem Wirkungsgrad und trotzdem geringem Steuerungsaufwand erreicht werden kann.

Die Aufgabe wird für die Steuerungsvorrichtung durch den Gegenstand des unabhängigen Patentanspruchs 1 gelöst. Vorteilhafte Weiterbildungen der Erfindung sind Gegenstand der Unteransprüche. Die Aufgabe wird ferner durch ein System gemäß dem unabhängigen Anspruch 6 gelöst.

Die erfindungsgemäße ophthalmochirurgische Steuerungsvorrichtung weist einen Frequenzgenerator auf, wobei der Frequenzgenerator aufweist:

- ein erstes Frequenzmodul, welches eingerichtet ist, ein erstes Schwingungssignal mit einer ersten Frequenz zu erzeugen, wobei die erste Frequenz niedriger als eine Ultraschallresonanzfrequenz eines an die Steuerungsvorrichtung anschließbaren ophthalmochirurgischen Piezohandstückes zur Emulsifikation einer Augenlinse ist, und

- ein zweites Frequenzmodul, welches eingerichtet ist, ein zweites Schwingungssignal mit einer zweiten Frequenz zu erzeugen, wobei die zweite Frequenz höher als die Ultraschallresonanzfrequenz des an die Steuerungsvorrichtung anschließbaren ophthalmochirurgischen Piezohandstückes ist,

wobei die Steuerungsvorrichtung ein Frequenzgenerator-Steuerungsmodul zur Ansteuerung des erstes Frequenzmoduls und des zweiten Frequenzmoduls aufweist.

Mit einer solchen Steuerungsvorrichtung kann ein erstes Schwingungssignal mit einer ersten Frequenz und ein zweites Schwingungssignal mit einer zweiten Frequenz erzeugt werden, wobei die erste Frequenz ungleich der zweiten Frequenz ist. Werden beide Frequenzen miteinander kombiniert, zum Beispiel durch eine additive Überlagerung, kann eine Schwebungsfrequenz erzeugt werden. Charakteristisch für eine solche Schwebungsfrequenz ist die sich ständig ändernde Amplitude. Besitzen das erste Schwingungssignal und das zweite Schwingungssignal eine Amplitude mit dem gleichen Betrag, führt eine Addition der beiden Schwingungssignale dazu, dass sich in regelmäßigen Abständen die Beträge der Amplituden zu Null addieren.

Die Amplitude der Schwebungsschwingung ändert sich mit einer Frequenz gemäß folgender Gleichung:

$$f_S = (f_1 - f_2) / 2$$

wobei $f_1$ die erste Frequenz des ersten Schwingungssignals und $f_2$ die zweite Frequenz des zweiten Schwingungssignals ist. Die sich regelmäßig zu Null addierenden Beträge der Schwebungsschwingung führen dazu, dass die Hohlnadel in diesen Momenten ruht und keine Energie in das Auge einführt. Wird die Hohlnadel mit einer solchen Schwebungsschwingung betrieben, kommt es ohne weitere Steuerung zu einer regelmäßigen Unterbrechung der in das Auge zugeführten Energie.

Es wird darauf hingewiesen, dass nicht vorrangig das Ziel besteht, eine vollständige Schwingungsunterbrechung zu erreichen, bei der die Amplitude der Schwingung einen Betrag von Null besitzt. Vielmehr geht es darum, während der Phakoemulsifikation immer wieder Zeiträume vorzusehen, in denen eine Abkühlung des Auges möglich ist. Dies wird auch dann erreicht, wenn in vorbestimmten Zeiträumen nur wenig Energie in das Auge zugeführt wird. Eine solche Situation liegt bei einer Schwingung vor, die eine relativ niedrige Amplitude besitzt. Je nach Wahl der beiden Schwingungsfrequenzen ergeben sich somit Zeiträume mit einer vorbestimmten Länge, in denen die Amplitude noch relativ niedrig ist.

Es wird ferner darauf hingewiesen, dass im Rahmen dieser Schrift mit der Ultraschallresonanzfrequenz des Piezohandstückes nicht nur die Resonanzfrequenz des Handstückes alleine, also mit einem Gehäuse, Piezostapel und damit gekoppelter Hohlnadel sowie elektrischen Anschlusselementen innerhalb des Gehäuses verstanden werden soll. Zusätzlich zu den genannten Komponenten sollen auch zum Beispiel ein Transformator, elektrischer Filter und andere für den Ultraschall-Schwingkreis erforderlichen elektrischen Bauelemente mit umfasst sein, da erst sämtliche Bauteile zusammen die Ultraschallresonanzfrequenz des Piezohandstückes bedingen.

Das erste Schwingungssignal kann auf einen ersten Stapel piezokeramischer Elemente im Piezohandstück aufgeprägt werden, während das zweite Schwingungssignal auf einen dahinter platzierten zweiten Stapel piezokeramischer Elemente des Piezohandstückes aufgeprägt werden kann. Die piezokeramischen Elemente sind somit mechanisch in Reihe geschaltet, so dass sich die Schwingungssignale additiv überlagern können. Eine aktive periodische Unterbrechung des Schwingungssignals, wie dies bei Lösungen gemäß dem Stand der Technik noch üblich ist, muss also beim Einsatz der erfindungsgemäßen Steuerungsvorrichtung nicht mehr vorgenommen werden. Vielmehr stellt sich bei einer additiven Überlagerung beider Schwingungssignale eine regelmäßige Unterbrechung von selbst ein.

Ein weiterer Vorteil dieser Ausführungsform besteht darin, dass zum Betrieb eines Piezohandstückes mit einem ersten Stapel und einem zweiten Stapel die Steuerungsvorrichtung mit zwei Verstärkern versehen werden kann, welche im Vergleich zu üblichen Verstärkern zum Betrieb eines Piezohandstückes nur die halbe Leistung aufweisen müssen. Dies verringert den Aufwand zum Betrieb des Piezohandstückes.

Außerdem kann durch entsprechend geschickte Wahl der ersten Frequenz und/oder der zweiten Frequenz der Betrieb des Piezohandstückes in der Resonanzfrequenz oder nahe der Resonanzfrequenz erreicht werden. Bei der Erfindung wird also ganz bewusst darauf verzichtet, eine aufwändige Messung des Spannungs- und Stromverlaufs über die Zeit und eine entsprechende Auswertung durch Ermittlung des Phasenwinkels vorzunehmen und daraufhin mit Verzögerung entsprechend nachzuregeln. Stattdessen erfolgt gar keine Regelung in Richtung zu einer Resonanzfrequenz. Es hat sich gezeigt, dass durch geschickte Wahl der ersten Frequenz und zweiten Frequenz ein Betrieb des Piezohandstückes in ausreichender Nähe zur momentanen Resonanzfrequenz möglich ist.

Erfindungsgemäß weist die Steuerungsvorrichtung ein Überlagerungsmodul auf, welches eingerichtet ist, das erste Schwingungssignal und das zweite Schwingungssignal additiv zu überlagern und als Überlagerungsschwingungssignal für den Betrieb des Piezohandstückes bereitzustellen. Damit ist es möglich, das Überlagerungsschwingungssignal als einziges Schwingungssignal von der Steuerungsvorrichtung einem Piezohandstück zur Verfügung zu stellen. Dies ermöglicht, dass jedes bisher bekannte Piezohandstück an eine solche Steuerungsvorrichtung angeschlossen werden kann.

Ferner ist es möglich, dass die Steuerungsvorrichtung eingerichtet ist, das erste Schwingungssignal und/oder das zweite Schwingungssignal nur während eines vorbestimmten Zeitraumes gleichzeitig bereitzustellen. Dies erlaubt den Betrieb mit einer ersten Frequenz, wobei zeitweise auch eine Schwebungsfrequenz vorliegt, so dass insgesamt eine sehr variable Frequenz des Piezohandstückes mit einer sehr variablen Amplitude vorliegt. Der Vorteil besteht darin, dass auch unterschiedlich harte und unterschiedlich große Fragmente einer Augenlinse zuverlässig zertrümmert werden können. Die Variabilität in der Frequenz und der Amplitude führt zu ganz unterschiedlich starken Energieeinträgen.

Vorzugsweise besitzt das erste Schwingungssignal eine erste Amplitude und das zweite Schwingungssignal eine zweite Amplitude, wobei die erste Amplitude höher als die zweite Amplitude ist oder die zweite Amplitude höher als die erste Amplitude ist. Damit entsteht eine sogenannte unreine Schwebung, bei der es keine sich zu Null addierende resultierende Amplitude der Gesamtschwingung ergibt. Auch dies kann zu einer großen Schwankung der zugeführten Energie führen, so dass die Wahrscheinlichkeit höher ist, dass Fragmente mit unterschiedlicher Härte und unterschiedlicher Größe zuverlässig und in kurzer Zeit zertrümmert werden können.

Gemäß einer bevorzugten Ausführungsform variiert die erste Frequenz und die zweite Frequenz zeitlich um einen vorbestimmten Betrag. Bei einer Addition der entsprechenden Schwingungssignale mit einer solchen ersten Frequenz und zweiten Frequenz kommt es zu keiner konstanten Schwebungsfrequenz, sondern zu einer Schwingung mit einer variierenden Frequenz. Die Variation der Frequenz erfolgt um den halben Betrag, so dass bei genügend großem Betrag die Hohlnadel des Handstückes immer wieder in der Resonanzfrequenz des Handstückes schwingen kann, ohne dass die Resonanzfrequenz genau gemessen oder eingestellt werden muss. Damit lässt sich ein sehr guter Wirkungsgrad erreichen. Durch die Addition der beiden Schwingungssignale kommt es ferner zu dem oben beschriebenen Effekt, dass sich die Amplituden in regelmäßigen Abständen zu Null addieren, so dass keine aktive Unterbrechung der Schwingungssignale erforderlich ist.

Die Aufgabe wird auch durch ein ophthalmochirurgisches System mit einer ophthalmochirurgischen Steuerungsvorrichtung wie vorstehend beschrieben gelöst, wobei das System ferner ein Piezohandstück, eine Fluidik-Steuerungsvorrichtung, eine Energieversorgungseinheit und eine Eingabeeinheit aufweist, wobei diese Komponenten zusammen mit einer zentralen Steuerungseinheit gekoppelt sind.

Weitere Vorteile und Merkmale der Erfindung werden mit Bezug auf die nachfolgenden Zeichnungen erklärt, in welchen zeigen:

Figur 1      eine schematische Darstellung einer ersten Ausführungsform einer ophthalmochirurgischen Steuerungsvorrichtung gemäß der Erfindung;

Figur 2      eine schematische Darstellung einer zweiten Ausführungsform der ophthalmochirurgischen Steuerungsvorrichtung gemäß der Erfindung;

Figur 3A - 3C      eine schematische Darstellung von einem ersten Schwingungssignal, einem zweiten Schwingungssignal und einem zugehörigen Überlagerungsschwingungssignal;

Figur 4A - 4C      eine weitere schematische Darstellung mit einem ersten Schwingungssignal und einem zweiten Schwingungssignal und einem zugehörigen Überlagerungsschwingungssignal;

Figur 5A - 5C      eine weitere Darstellung eines ersten Schwingungssignals und eines zweiten Schwingungssignals und eines zugehörigen Überlagerungsschwingungssignals;

Figur 6A - 6C      eine weitere Darstellung eines ersten Schwingungssignals und eines zweiten Schwingungssignals und eines zugehörigen Überlagerungsschwingungssignals;

Figur 7A - 7C      eine schematische Darstellung des ersten Schwingungssignals und des zweiten Schwingungssignals und des zugehörigen Überlagerungsschwingungssignals gemäß Figur 6; und

Figur 8      eine schematische Darstellung des ophthalmochirurgischen Systems gemäß der Erfindung.

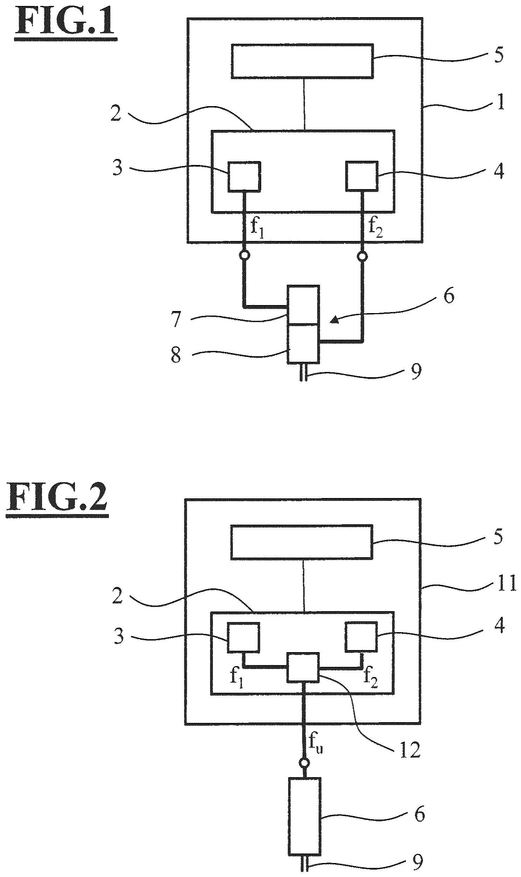

In Figur 1 ist eine ophthalmochirurgische Steuerungsvorrichtung 1 gemäß der Erfindung dargestellt. Die Steuerungsvorrichtung weist einen ophthalmochirurgischen Frequenzgenerator 2 mit einem ersten Frequenzmodul 3 und einem zweiten Frequenzmodul 4 auf. Das erste Frequenzmodul 3 ist eingerichtet, ein erstes Schwingungssignal mit einer ersten Frequenz $f_1$ zu erzeugen, wobei das zweite Frequenzmodul 4 eingerichtet ist, ein zweites Schwingungssignal mit einer zweiten Frequenz $f_2$ zu erzeugen. Die Ansteuerung des ophthalmochirurgischen Frequenzgenerators 2 erfolgt mittels eines Frequenzgenerator-Steuermoduls 5.

Bei dieser Ausführungsform der Steuerungsvorrichtung 1 wird am Ausgang der Steuerungsvorrichtung das erste Schwingungssignal mit der ersten Frequenz $f_1$ und das zweite Schwingungssignal mit der zweiten Frequenz $f_2$ bereitgestellt. Beide Schwingungssignale können einem Piezohandstück 6 zugeführt werden, wobei das erste Schwingungssignal einem ersten Piezostapel 7 und das zweite Schwingungssignal einem zweiten Piezostapel 8 zugeführt werden können. Beide Piezostapel sind in Reihe angeordnet, so dass sich die Signale additiv überlagern können. Damit ist es möglich, von beiden Schwingungssignalen eine Schwebungsfrequenz zu erzeugen, so dass die Hohlnadel 9 des Piezohandstückes 6 mit einer Schwebungsfrequenz schwingen kann.

Figur 2 zeigt eine zweite Ausführungsform einer ophthalmochirurgischen Steuerungsvorrichtung 11, wobei die Steuerungsvorrichtung einen ophthalmochirurgischen Frequenzgenerator 2 mit einem ersten Frequenzmodul 3 und einem zweiten Frequenzmodul 4 aufweist. Wie bei der ersten Ausführungsform der ophthalmochirurgischen Steuerungsvorrichtung 1 kann das erste Frequenzmodul 3 ein erstes Schwingungssignal mit einer ersten Frequenz $f_1$ und das zweite Frequenzmodul 4 ein zweites Schwingungssignal mit einer zweiten Frequenz $f_2$ abgeben. Bei dieser zweiten Ausführungsform der ophthalmochirurgischen Steuerungsvorrichtung 11 werden jedoch beide Schwingungssignale einem Überlagerungsmodul 12 zugeführt, welches eingerichtet ist, das erste Schwingungssignal mit der ersten Frequenz $f_1$ und das zweite Schwingungssignal mit der zweiten Frequenz $f_2$ additiv zu überlagern und als Überlagerungsschwingungssignal mit einer Frequenz $f_u$ am Ausgang der Steuerungsvorrichtung 11 bereitzustellen. Dieses Überlagerungsschwingungssignal kann dann einem Piezohandstück 6 zugeführt werden, wobei dieses Piezohandstück nur einen einzigen Anschluss zum Betrieb sämtlicher Piezostapel innerhalb des Piezohandstückes benötigt. Ein Vorteil dieser Ausführungsform besteht darin, dass jedes herkömmliche Piezohandstück mit nur einem Anschluss genutzt werden kann.

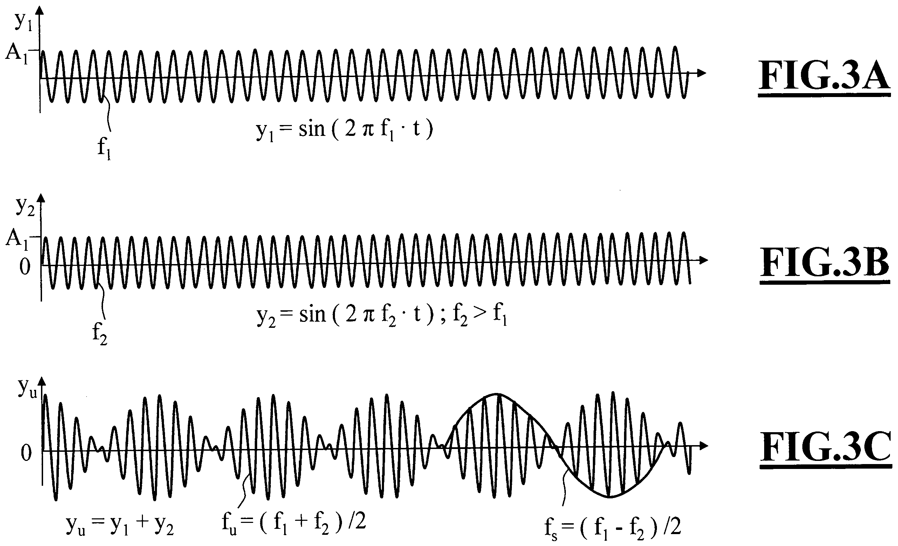

In Figur 3A ist eine schematische Darstellung eines ersten Schwingungssignals mit einer ersten Frequenz $f_1$ mit einer Amplitude $A = 1$ dargestellt. Bei dem ersten Schwingungssignal handelt sich um eine Sinusschwingung gemäß der Gleichung $y_1 = \sin(2\pi f_1 \cdot t)$. Ferner ist in Figur 3B ein zweites Schwingungssignal mit einer zweiten Frequenz $f_2$ und einer Amplitude $A = 1$ dargestellt. Das zweite Schwingungssignal ist eine Sinusschwingung gemäß der Gleichung $y_2 = \sin(2\pi f_2 \cdot t)$, wobei $f_2$ nicht gleich $f_1$ ist. Werden beide Schwingungssignale überlagert, ergibt sich ein Überlagerungsschwingungssignal gemäß der Gleichung $y_u = y_1 + y_2$, siehe Figur 3C, wobei das Überlagerungsschwingungssignal eine Überlagerungsfrequenz $f_u = (f_1 + f_2) / 2$ mit einer Amplitudenfrequenz $f_s = (f_1 - f_2) / 2$ besitzt.

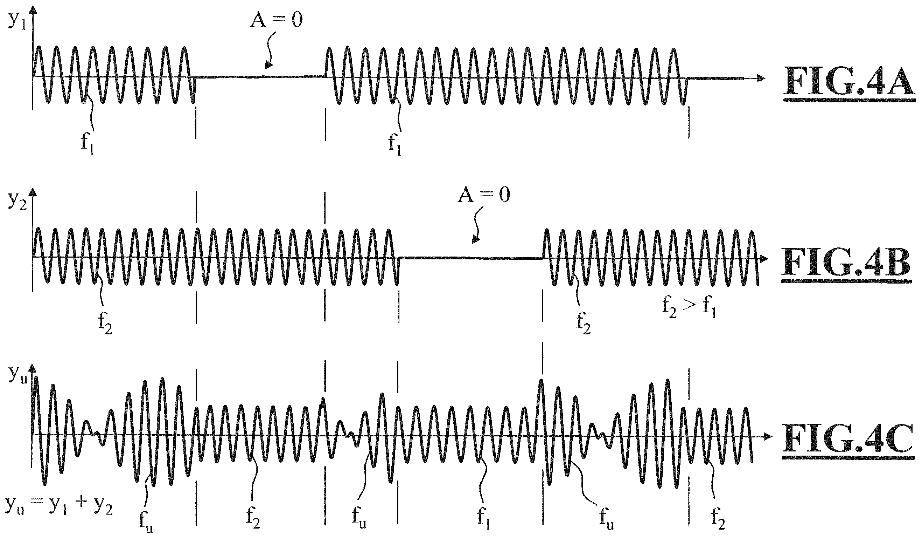

In Figur 4A ist eine weitere Darstellung eines ersten Schwingungssignales mit einer ersten Frequenz $f_1$ dargestellt; Fig. 4B zeigt ein zweites Schwingungssignal mit einer zweiten Frequenz $f_2$. Im Gegensatz zu den in Fig. 3A und Fig. 3B dargestellten Schwingungssignalen besitzt das erste Schwingungssignal gemäß Figur 4A für einen vorbestimmten Zeitraum eine Amplitude $A = 0$. Das zweite Schwingungssignal mit einer Frequenz $f_2$ besitzt ebenfalls einen Zeitraum, in welchem die Amplitude $A = 0$ ist, siehe Fig. 4B. Bei einer Überlagerung der beiden Schwingungssignale ergibt sich ein Überlagerungsschwingungssignal mit unterschiedlichen Frequenzen, siehe Fig. 4C. Bei der in Fig. 4C dargestellten Ausführungsform tritt zuerst eine Überlagerungsfrequenz $f_u$, dann die zweite Frequenz $f_2$, dann wieder die Überlagerungsfrequenz $f_u$, dann die Frequenz $f_1$, dann die Überlagerungsfrequenz $f_u$, und dann die zweite Frequenz $f_2$ auf. Wie deutlich zu erkennen ist, ergibt dies eine Schwingung mit einer stark schwankenden Amplitude und schwankenden Frequenzen, so dass eine hohe Wahrscheinlichkeit besteht, dass Linsenfragmente mit unterschiedlicher Härte und unterschiedlicher Größe zuverlässig und in kurzer Zeit zertrümmert werden können.

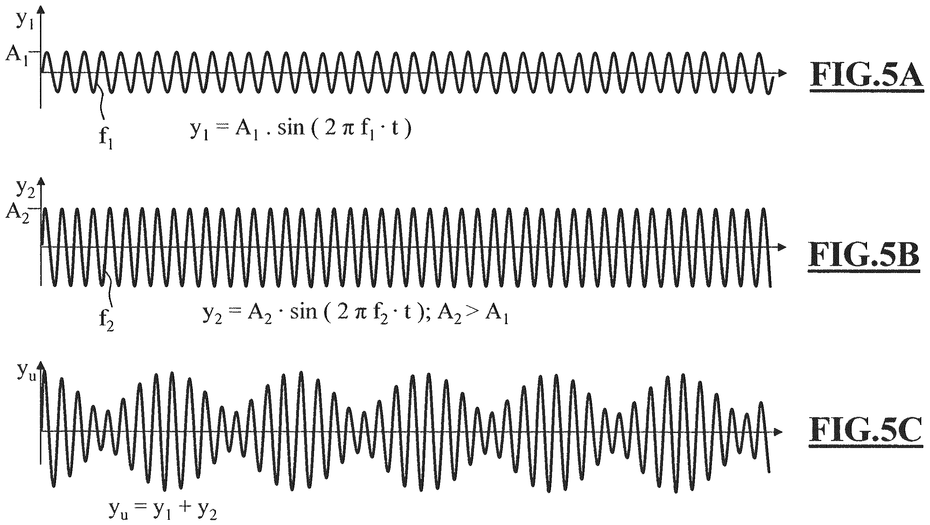

In Fig. 5A ist eine weitere Darstellung eines ersten Schwingungssignals mit einer ersten Frequenz $f_1$ und einer Amplitude $A_1$ dargestellt. Fig. 5B zeigt ein zweites Schwingungssignal mit einer zweiten Frequenz $f_2$ und einer zweiten Amplitude $A_2$, wobei $f_1$ nicht gleich $f_2$ und $A_1$ nicht gleich $A_2$ ist. Bei einer additiven Überlagerung beider Schwingungssignale ergibt sich ein Überlagerungsschwingungssignal, siehe Fig. 5C, bei der es keine sich zu Null addierende Amplitude der Schwingung gibt. Durch geschickte Wahl der Amplituden $A_1$ und $A_2$ sowie der Frequenzen $f_1$ und $f_2$ kann ein Überlagerungsschwingungssignal erzeugt werden, welches genügend Energie zum Zertrümmern von unterschiedlich großen und harten Linsenpartikeln zur Verfügung stellt, jedoch auch genügend Zeiträume aufweist, in denen die Energie so gering ist, dass sich eine übermäßig starke Erwärmung des Auges verhindern lässt.

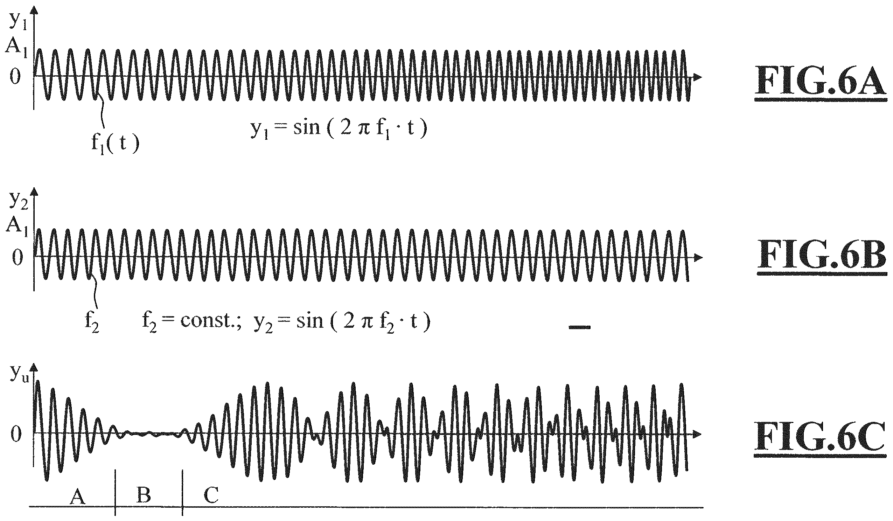

In Fig. 6A ist eine weitere Darstellung mit einem ersten Schwingungssignal mit einer ersten Frequenz und in Fig. 6B ein zweites Schwingungssignal mit einer zweiten Frequenz dargestellt, welche oberhalb der Resonanzfrequenz liegt. Das erste Schwingungssignal besitzt eine Frequenz $f_1$, welche zeitlich variiert und nicht konstant ist. Das zweite Schwingungssignal besitzt eine Frequenz $f_2$, welche konstant ist. Durch additive Überlagerung beider Schwingungssignale ergibt sich ein Überlagerungsschwingungssignal mit einer Überlagerungsfrequenz, siehe Fig. 6C. Im Bereich "A" ist $f_2$ größer als $f_1(t)$, so dass eine Überlagerungsfrequenz entsteht, welche kleiner als die Resonanzfrequenz des Piezohandstückes ist. Im Bereich "B" ist $f_1$ nahezu gleich $f_2$, wobei die Schwingungen nahezu um 180 Grad phasenverschoben sind. Im Bereich "C" ist $f_1(t)$ größer als $f_2$, so dass die Überlagerungsfrequenz größer als die Resonanzfrequenz des Piezohandstückes ist. Im Bereich "A" muss es eine Zone geben, in der genau die Resonanzfrequenz des Handstückes erreicht ist. Die Überlagerungsfrequenz schwankt also kontinuierlich, so dass immer sichergestellt ist, dass das Handstück für einen gewissen Zeitraum genau innerhalb der Resonanzfrequenz betrieben wird, obwohl keine aufwendige Messung eines Spannungs- und Stromverlaufes innerhalb des Piezohandstückes erforderlich ist.

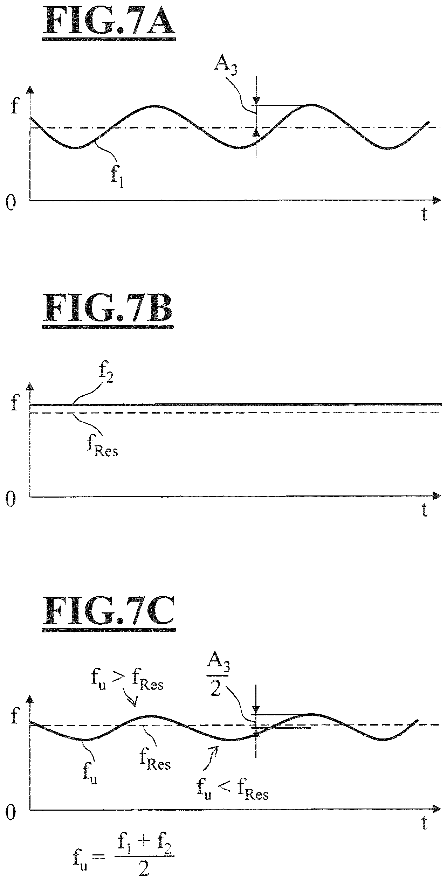

In Figur 7 ist diese Situation nochmals erläutert. Das erste Schwingungssignal mit der ersten Frequenz schwankt sinusförmig mit einer Frequenz $f_1(t)$ und einer Amplitude $A_3$, siehe Fig. 7A. Das zweite Schwingungssignal besitzt eine konstante Frequenz $f_2$, welche höher als die Resonanzfrequenz $f_{Res}$ des Piezohandstückes ist, siehe Fig. 7B. Bei einer additiven Überlagerung des ersten Schwingungssignals mit dem zweiten Schwingungssignal ergibt sich ein Überlagerungsschwingungssignal mit einer Frequenz $f_u$, welche ebenfalls sinusförmig variiert. Bei entsprechender Wahl der Frequenz $f_1$ und $f_2$ und der Amplitude $A_3$ ist es möglich, dass das Überlagerungsschwingungssignal eine Frequenz besitzt, welche ständig um die Resonanzfrequenz des Piezohandstückes schwankt. Damit gibt es Bereiche, in denen das Überlagerungsschwingungssignal eine Überlagerungsfrequenz besitzt, welche größer als die Resonanzfrequenz des Handstückes ist und welcher kleiner als die Resonanzfrequenz des Handstückes ist.

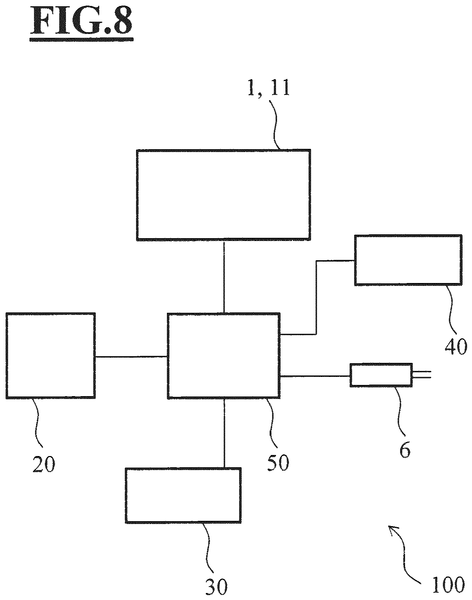

In Figur 8 ist eine schematische Darstellung des ophthalmochirurgischen Systems 100 gemäß der Erfindung dargestellt, wobei das System aufweist: eine ophthalmochirurgische Steuerungsvorrichtung 1 oder 11, eine Fluid-Steuerungsvorrichtung 20, eine Energieversorgungseinheit 30, eine Eingabeeinheit 40, ein Piezo-Handstück 6, wobei diese Komponenten zusammen mit einer zentralen Steuerungseinheit 50 gekoppelt sind.

**Patentansprüche**

1. Ophthalmochirurgisches System (100), umfassend eine ophthalmochirurgische Steuerungsvorrichtung (1, 11), ein Piezohandstück (6), eine Fluidik-Steuerungsvorrichtung (20), eine Energieversorgungseinheit (30), eine Eingabeeinheit (40), und eine zentrale Steuerungseinheit (50), wobei diese Komponenten zusammen mit der zentralen Steuerungseinheit (50) gekoppelt sind, wobei die ophthalmochirurgische Steuerungsvorrichtung (1,11) einen Frequenzgenerator (2) aufweist, wobei der Frequenzgenerator aufweist:

   - ein erstes Frequenzmodul, (3) welches eingerichtet ist, ein erstes Schwingungssignal mit einer ersten Frequenz $f_1$ zu erzeugen, wobei die erste Frequenz $f_1$ niedriger als die Ultraschallresonanzfrequenz des an die Steuerungsvorrichtung (1,11) angeschlossenen Piezohandstückes (6) zur Emulsifikation einer Augenlinse ist, und das erste Schwingungssignal eine Sinusschwingung gemäß der Gleichung $y_1 = \sin(2\pi f_1 \cdot t)$ ist, und
   - ein zweites Frequenzmodul (4), welches eingerichtet ist, ein zweites Schwingungssignal mit einer zweiten Frequenz $f_2$ zu erzeugen, wobei die zweite Frequenz $f_2$ höher als die Ultraschallresonanzfrequenz des an die Steuerungsvorrichtung angeschlossenen Piezohandstückes (6) ist, und das zweite Schwingungssignal eine Sinusschwingung gemäß der Gleichung $y_2 = \sin(2\pi f_2 \cdot t)$ ist,

   und wobei die Steuerungsvorrichtung (1,11) ein Frequenzgenerator-Steuerungsmodul (5) zur Ansteuerung des ersten Frequenzmoduls und des zweiten Frequenzmoduls aufweist und wobei die Steuerungsvorrichtung (1,11) ein Überlagerungsmodul (12) aufweist, welches eingerichtet ist, das erste Schwingungssignal und das zweite Schwingungssignal additiv zu überlagern und als Überlagerungs-Schwingungssignal gemäß der Gleichung $y_u = y_1 + y_2$ für den Betrieb des Piezohandstückes (6) bereitzustellen, so dass eine Schwebungsschwingung mit einer Schwebungsfrequenz erzeugt wird.

2. Steuerungsvorrichtung nach Anspruch 1, wobei die Steuerungsvorrichtung (1,11) eingerichtet ist, das erste Schwingungssignal und/oder das zweite Schwingungssignal nur während eines vorbestimmten Zeitraumes gleichzeitig bereitzustellen.

3. Steuerungsvorrichtung nach einem der vorherigen Ansprüche, wobei das erste Schwingungssignal eine erste Amplitude und das zweite Schwingungssignal eine zweite Amplitude besitzt, wobei die erste Amplitude höher als die zweite Amplitude ist oder die zweite Amplitude höher als die erste Amplitude ist.

4. Steuerungsvorrichtung nach einem der vorherigen Ansprüche, wobei die erste Frequenz $f_1$ und/oder die zweite Frequenz $f_2$ um einen vorbestimmten Betrag zeitlich variiert.

**Claims**

1. Ophthalmic surgical system (100) comprising an ophthalmic surgical control device (1, 11), a piezoelectric handpiece (6), a fluidics control device (20), a power supply unit (30), an input unit (40), and a central control unit (50), wherein these components together are coupled to the central control unit (50), wherein the ophthalmic surgical control device (1, 11) comprises a frequency generator (2), wherein the frequency generator comprises:

   - a first frequency module (3) configured to produce a first vibration signal at a first frequency $f_1$, wherein the first frequency $f_1$ is lower than the ultrasonic resonant frequency of the piezoelectric handpiece (6), for emulsifying an eye lens, that is connected to the control device (1, 11), and the first vibration signal is a sinusoidal vibration according to the equation $y_1 = \sin(2\pi f_1 \cdot t)$, and
   - a second frequency module (4) configured to produce a second vibration signal at a second frequency $f_2$, wherein the second frequency $f_2$ is higher than the ultrasonic resonant frequency of the piezoelectric handpiece (6) that is connected to the control device, and the second vibration signal is a sinusoidal vibration according to the equation $y_2 = \sin(2\pi f_2 \cdot t)$,

   and wherein the control device (1, 11) comprises a frequency generator control module (5) for actuating the first frequency module and the second frequency module, and wherein the control device (1, 11) comprises a superposition module (12) configured to superpose, in an additive manner, the first vibration signal and the second vibration signal and to provide this as superposition vibration signal according to the equation $y_u = y_1 + y_2$ for operating the piezoelectric handpiece (6), so that a beat vibration having a beat frequency is produced.

2. Control device according to Claim 1, wherein the control device (1, 11) is configured to provide the first vibration signal and/or the second vibration signal simultaneously only during a predetermined period of time.

3. Control device according to one of the preceding claims, wherein the first vibration signal has a first amplitude and the second vibration signal has a second amplitude, wherein the first amplitude is higher than the second amplitude or the second amplitude is higher than the first amplitude.

4. Control device according to one of the preceding claims, wherein the first frequency $f_1$ and/or the second frequency $f_2$ vary in time by a predetermined absolute value.

**Revendications**

1. Système ophtalmologique (100), comprenant un dispositif de commande chirurgical ophtalmologique (1, 11), une pièce piézoélectrique à main (6), un dispositif de commande fluidique (20), une unité d'alimentation électrique (30), une unité d'entrée (40) et une unité de commande centrale (50), ces composants étant accouplés conjointement à unité de commande centrale (50), le dispositif de commande chirurgical ophtalmologique (1, 11) étant un générateur de fréquence (2), le générateur de fréquence comprenant :

   - un premier module de fréquence (3) conçu pour générer un premier signal oscillant d'une première fréquence $f_1$, la première fréquence $f_1$ étant inférieure à la fréquence de résonance ultrasonore de la pièce piézoélectrique à main (6) raccordée au dispositif de commande (1, 11) pour émulsifier un cristallin, et le premier signal oscillant étant une oscillation sinusoïdale selon l'équation $y_1 = \sin(2\pi f_1 \cdot t)$, et
   - un deuxième module de fréquence (4) conçu pour générer un deuxième signal oscillant d'une deuxième fréquence $f_2$, la deuxième fréquence $f_2$ étant supérieure à la fréquence de résonance ultrasonore de la pièce piézoélectrique (6) raccordée au dispositif de commande, et le deuxième signal oscillant étant une oscillation sinusoïdale selon l'équation $y_2 = \sin(2\pi f_2 \cdot t)$,

   et le dispositif de commande (1, 11) comportant un module de commande de générateur de fréquence (5) destiné à commander le premier module de fréquence et le deuxième module de fréquence, et le dispositif de commande (1, 11) comprenant un module de superposition (12) conçu par superposer le premier signal d'oscillation et le deuxième signal d'oscillation de manière additive et pour les produire sous la forme d'un signal d'oscillation de superposition selon l'équation $y_u = y_1 + y_2$ pour le fonctionnement de la pièce piézoélectrique à main (6) de manière à générer une oscillation de battement avec une fréquence de battement.

2. Dispositif de commande selon la revendication 1, le dispositif de commande (1, 11) étant conçu pour produire simultanément le premier signal d'oscillation et/ou le deuxième signal d'oscillation uniquement pendant un intervalle de temps prédéterminé.

3. Dispositif de commande selon l'une des revendications précédentes, le premier signal d'oscillation ayant une première amplitude et le deuxième signal d'oscillation ayant une deuxième amplitude, la première amplitude étant supérieure à la deuxième amplitude ou la deuxième amplitude étant supérieure à la première amplitude.

4. Dispositif de commande selon l'une des revendications précédentes, la première fréquence $f_1$ et/ou la deuxième fréquence $f_2$ variant dans le temps d'une valeur prédéterminée.

## FIG.1

## FIG.2

$$y_1 = \sin(2\pi f_1 \cdot t)$$

**FIG.3A**

$$y_2 = \sin(2\pi f_2 \cdot t)\; ; f_2 > f_1$$

**FIG.3B**

$$y_u = y_1 + y_2 \qquad f_u = (f_1 + f_2)/2 \qquad f_s = (f_1 - f_2)/2$$

**FIG.3C**

$A = 0$

$y_1$

$f_1$     $f_1$

**FIG.4A**

$y_2$

$A = 0$

$f_2$     $f_2$     $f_2 > f_1$

**FIG.4B**

$y_u$

$y_u = y_1 + y_2$

$f_u$    $f_2$    $f_u$    $f_1$    $f_u$    $f_2$

**FIG.4C**

EP 2 760 398 B1

EP 2 760 398 B1

$$y_1 = A_1 \cdot \sin(2\pi f_1 \cdot t)$$

**FIG.5A**

$$y_2 = A_2 \cdot \sin(2\pi f_2 \cdot t); \ A_2 > A_1$$

**FIG.5B**

$$y_u = y_1 + y_2$$

**FIG.5C**

$$y_1 = \sin(2\pi f_1 \cdot t)$$

$f_1(t)$

**FIG.6A**

$$f_2 = \text{const.}; \quad y_2 = \sin(2\pi f_2 \cdot t)$$

$f_2$

**FIG.6B**

A | B | C

**FIG.6C**

EP 2 760 398 B1

# FIG.7A

# FIG.7B

# FIG.7C

$$f_u = \frac{f_1 + f_2}{2}$$

# FIG.8

1, 11

20

30

50

6

40

100

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1849444 A1
- EP 0765637 A

- WO 2010054144 A2
- US 6997935 B2